# EUROPEAN PATENT SPECIFICATION

(11) **EP 3 493 848 B1**
(45) Date of publication and mention of the grant of the patent: **29.09.2021**
(21) Application number: 17754626.4
(22) Date of filing: 31.07.2017
(51) Int. Cl.: A61K 31/4184, A61K 45/06, A61P 43/00

(54) **HETEROCYCLIC DIAMIDINES**
HETEROCYCLISCHE DIAMIDINE
DIAMIDINES HÉTÉROCYCLIQUES

(30) Priority: 02.08.2016 EP 16182475
(43) Date of publication of application: 12.06.2019
(73) Proprietor: Friedrich-Alexander-Universität Erlangen-Nürnberg, 91054 Erlangen (DE); Universitätsklinikum Erlangen, 91054 Erlangen (DE)
(72) Inventor: RAMMING, Andreas, 91052 Erlangen (DE); DISTLER, Jörg, 91090 Effeltrich (DE); SCHETT, Georg, 91054 Erlangen (DE); WOHLFAHRT, Thomas, 91054 Erlangen (DE)
(74) Representative: Vossius & Partner Patentanwälte Rechtsanwälte mbB
(86) International application number: PCT/EP2017/069276
(87) International publication number: WO 2018/024654

(56) References cited:
- WO-A2-2008/057556
- DE-A1- 2 711 362
- US-A- 5 817 687
- US-A1- 2006 083 682
- DOMINIQUE C. STEPHENS ET AL: "Pharmacologic efficacy of PU.1 inhibition by heterocyclic dications: a mechanistic analysis", NUCLEIC ACIDS RESEARCH, vol. 44, no. 9, 13 April 2016 (2016-04-13) , pages 4005-4013, XP055335545, ISSN: 0305-1048, DOI: 10.1093/nar/gkw229
- M. MUNDE ET AL: "Structure-dependent inhibition of the ETS-family transcription factor PU.1 by novel heterocyclic diamidines", NUCLEIC ACIDS RESEARCH, vol. 42, no. 2, 23 October 2013 (2013-10-23), pages 1379-1390, XP055335515, ISSN: 0305-1048, DOI: 10.1093/nar/gkt955
- BRENDLE J J ET AL: "ANTILEISHMANIAL ACTIVITIES OF SEVERAL CLASSES OF AROMATIC DICATIONS", ANTIMICROBIAL AGENTS AND CHEMOTHERAPY, AMERICAN SOCIETY FOR MICROBIOLOGY, US, vol. 46, no. 3, 1 March 2002 (2002-03-01), pages 797-807, XP001148797, ISSN: 0066-4804, DOI: 10.1128/AAC.46.3.797-807.2002

## Description

### Field of the invention

The present invention relates to pharmaceutical compositions comprising heterocyclic furamidine derivatives and their use in the prophylaxis and/or treatment of diseases associated with fibrosis, in particular of diseases such as systemic sclerosis (*limited cutaneous scleroderma* and *diffuse cutaneous scleroderma*)*,* pulmonary fibrosis, hepatic cirrhosis, renal fibrosis, chronic graft-versus-host disease, Crohn's disease, arthrofibrosis, myelofibrosis, Dupuytren's disease and nephrogenic systemic fibrosis and tumours selected from mamma carcinoma, endometrial adenocarcinoma, ovarian serous tumor, lung adenocarcinoma, lung squamous cell tumor, colorectal tumor and pancreatic. The heterocyclic furamidine derivatives, wherein the bridging phenyl groups of furamidine have been replaced with benzimidazol groups and optionally the central furan by selenophene, used in accordance with the present invention are inhibitors of the ETS-family transcription factor PU.1.

PU.1 inhibitors are inter alia defined in Munde et al., Nucleic Acids Res. 2014 Jan; 42(2):1379-90. doi: 10.1093/nar/gkt955. Epub 2013 Oct 23 and Stephens et al., Nucleic Acids Res. 2016 Apr: 1-9; doi: 10.1093/nar/gkw229.

### Background of the invention

The ETS family of transcription factors in mouse or humans is comprised of around 27 unique family members that contain an evolutionary conserved DNA-binding domain called the Ets domain that belongs to the winged helix-turn-helix DNA-binding domain superfamily. ETS transcription factors bind to the consensus DNA sequence [5'-GGA(A/T)-3']. Further DNA-binding specificity within ETS family members is determined by adjacent DNA sequences and through the binding of additional transcriptional partners. The location of the Ets domain is variable within ETS family members, also the expression pattern of ETS family members varies widely. ETS transcription factor target genes including oncogenes, tumour suppressor genes, and genes involved in apoptosis, differentiation, angiogenesis, and invasion and metastasis. Importantly, aberrant ETS family expression contributes to malignant transformation and tumour progression.

The ETS family includes both transcriptional activators and suppressors. The normal ETS transcription factors have been implicated in virtually all cellular processes, including growth, development, differentiation, survival and oncogenic transformation (Foos and Hauser, Transcription Factors, Eds Gossen et al, Springer (2004) 166:259-275; Seth and Watson Eur J Cancer 2005, Nov; 41(16): 2462-2478).

The fact that some ETS transcription factors are involved in malignant transformation and tumour progression makes them potential molecular targets for pharmacological control of gene regulation in particular for selective cancer therapy (Oikawa, Cancer Sci 2004, Aug; 9588): 626-633; Seth and Watson Eur J Cancer 2005, Nov; 41(16): 2462-2478; Turner and Watson Expert Review of Anticancer Therapy, 2008 8(1)).

The PU.1 gene encodes an Ets family transcription factor which controls expression of many B cell- and macrophage-specific genes. Expression of the gene is critical for development of lymphoid and myeloid cell lineages. Heterozygous mutations in PU.1 mostly disrupting the PU.1 DNA binding function have been identified in a number of acute myeloid leukemia patients (Foos and Hauser, Transcription Factors, Eds Gossen et al, Springer (2004) 166:259-275). Like all ETS proteins, PU.1 binds sequence specifically to 10-bp sites by inserting a recognition helix into the major groove of a 5'-GGAA-3'consensus, accompanied by contacts with the flanking minor groove (Munde et al., Nucleic Acids Research, 2014, Vol. 42, No. 2, 1379-1390).

Furamidine, a diamidine of formula 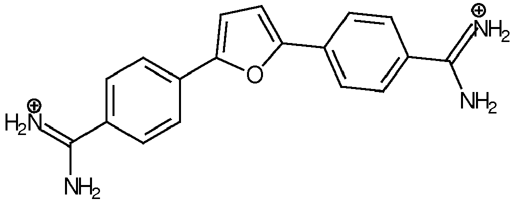 and heterocyclic furamidine derivatives have been disclosed to target a portion of the DNA recognition site of the ETS-family transcription factors ERG (Nhili et al., Nucleic Acids Research, 2013 Vol. 41, No.1) and PU.1 (Munde et al., Nucleic Acids Research, 2014, Vol. 42, No. 2, 1379-1390).

Fibrotic diseases impose a major socioeconomic burden on modern societies and account for up to 45% of deaths in the developed world (Wynn T.A., The Journal of Pathology 214, 199-210 (2008), Wynn and Ramalingam, Nature Medicine 18, 1028-1040 (2012)). Despite the great medical need, anti-fibrotic therapies are not yet available for clinical use (Wynn and Ramalingam, Nature Medicine 18, 1028-1040 (2012), Ramming et al, Pharmacological Research: the official journal of the Italian Pharmacological Society 100, 93-100 (2015)). Fibrosis may occur after defined noxious stimuli, but in many cases, no initiating trigger can be identified. Although fibro-proliferative responses are an indispensable part of wound healing, disturbances of tissue responses can culminate in excessive fibrosis and fibro-destructive disease (Wynn and Ramalingam, Nature Medicine 18, 1028-1040 (2012). Fibrosis is the final, common pathological outcome of many chronic inflammatory diseases including systemic sclerosis (SSc), rheumatoid arthritis, Crohn's disease, ulcerative colitis and myelofibrosis. Furthermore, dysregulated fibro-proliferative responses are directly and indirectly implicated in many very common diseases such as liver cirrhosis, kidney disease and heart failure, and influences tumor invasion and metastasis, chronic graft rejection and the pathogenesis of many myopathies (Wynn and Ramalingam, Nature Medicine 18, 1028-1040 (2012)). The histopathological feature of SSc is an excessive accumulation of extracellular matrix that often disrupts the physiological architecture of affected tissue. Fibroblasts are the principle source of extracellular matrix and have been accused as major culprits of fibrotic disorders (Varga and Abraham, The Journal of Clinical Investigation 117, 557-567 (2007)). Although the molecular mechanisms underlying the aberrant activation of fibroblasts in SSc and other fibrotic diseases have only partially been unraveled, there is considerable evidence that transforming growth factor-beta (TGF-β) is a key regulator of fibroblast activation. TGF-β signaling is activated in fibrotic diseases and fibroblasts display nuclear accumulation of the downstream mediator Smad3 and increased transcription of TGF-β target genes (Ramming et al, Pharmacological Research: the official journal of the Italian Pharmacological Society 100, 93-100 (2015), Whitfield et al., Proc Natl Acad Sci USA 100, 12319-12324 (2003)). Moreover, TGF-β potently activates fibroblasts and induces an expression profile in resting normal fibroblasts that is reminiscent of SSc fibroblasts (Whitfield et al., Proc Natl Acad Sci USA 100, 12319-12324 (2003)). The central role of TGF-β signaling is further highlighted by the development of a systemic fibrotic disease in mice with fibroblast-specific overexpression of constitutively active TGF-β receptor type I. However, the knowledge of the crucial role of TGF-β has not yet been translated into molecular therapies and effective targeted treatments for fibrosis in SSc and other fibrotic diseases are not available for clinical use (Ramming et al, Pharmacological Research: the official journal of the Italian Pharmacological Society 100, 93-100 (2015)).

Tumors are known as wounds that do not heal (Kalluri and Zeisberg, Nature Reviews. Cancer 6, 392-401(2006)). Cancer associated fibroblasts (CAFs) are associated with cancer cells at all stages of cancer progression, and their structural and functional contributions to this process are well known. This implies that CAFs have a prominent role in the progression, growth and spread of cancers and represent an important target for cancer therapies.

### Summary of the invention

The invention is defined by the claims.

Disclosed is a pharmaceutical composition comprising a PU.1 inhibitor for use in the prophylaxis or treatment of a disease associated with fibrosis. Moreover, the present invention provides a pharmaceutical composition comprising a compound of the general formula (I) 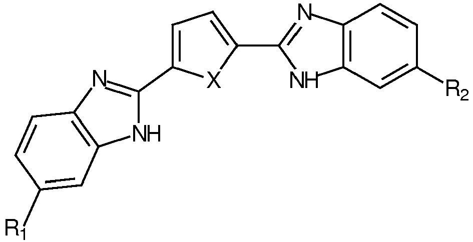
wherein X is Se, O, Te, S or NH,
and R₁ and R₂ are the same or different and are selected from 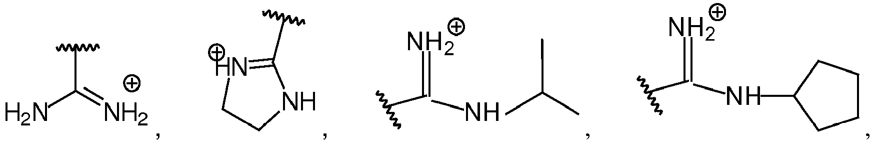 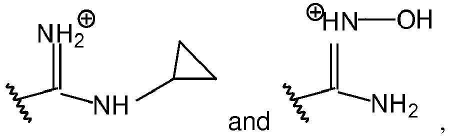
or a pharmaceutically acceptable salt thereof;
optionally comprising a carrier and one or more pharmaceutically acceptable excipients, for use in the prophylaxis or treatment of a disease associated with fibrosis.

In particular, the present invention provides a pharmaceutical composition comprising a compound of the general formula (I) 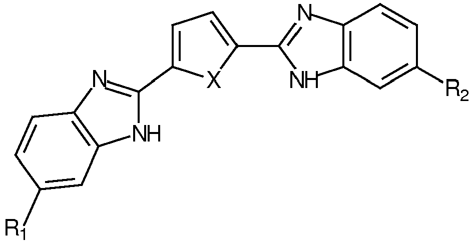
wherein X is Se or O and
R₁ and R₂ are the same or different and are selected from 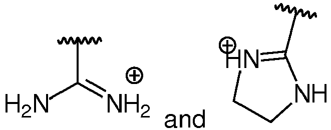
or a pharmaceutically acceptable salt thereof;
optionally comprising a carrier and one or more pharmaceutically acceptable excipients.

The compositions according to the present invention may be used in the prophylaxis and/or treatment of a disease associated with fibrosis.

Hereinafter, the compound of formula I, wherein X is selen and R₁ and R₂ are 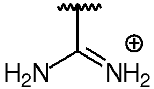 is referred to as DB1976, whereas the corresponding oxygen-containing derivative is referred to as DB270.

The compound of formula (I), wherein X is selen and R₁ and R₂ are 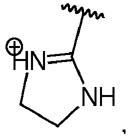 is referred to as DB1977.

The compositions according to the present invention may be used alone or in combination with a further pharmaceutically active ingredient such as an anti-proliferative agent or an anti-cancer agent or therapy, such as radiotherapy.

Thus, the present invention provides a pharmaceutical composition for use according to the present invention, which is to be administered in combination with at least one anti-proliferative agent.

Pharmaceutical composition for use according to the present invention, which is to be administered in combination with at least one anti-cancer agent and/or radiotherapy.

The problem underlying the present invention is to provide a therapy or prophylaxis for diseases associated with dysregulated fibro-proliferative responses such as systemic sclerosis, rheumatoid arthritis, Crohn's Disease, ulcerative colitis, myelofibrosis, hepatic cirrhosis, kidney disease, heart failure and other diseases associated with fibrosis (fibrotic diseases) and certain tumours such as breast cancer, pancreatic cancer and colon cancer.

### Detailed description of the invention

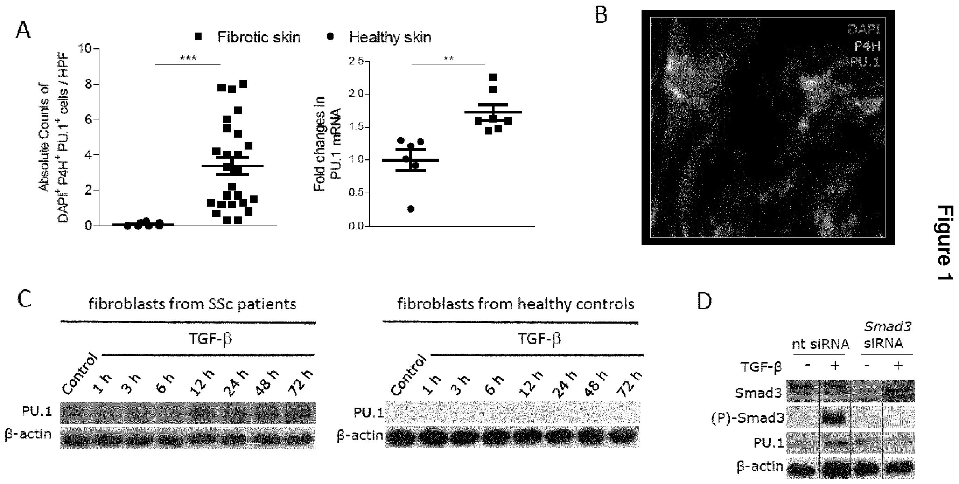

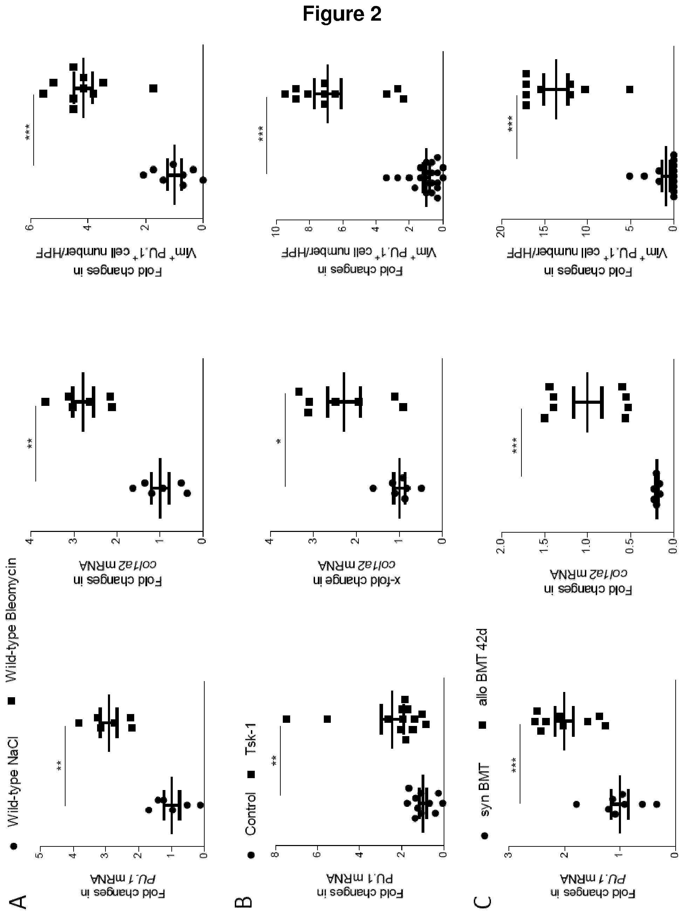

Inventors have identified Spi-1/PU.1 as a TGF-β target gene that is up-regulated in fibroblasts that are found in fibrotic tissues such as fibrotic skin of SSc patients **(****Fig. 1A****).** Confocal microscopy revealed upregulation of PU.1 in fibroblasts **(****Fig. 1B****).** In contrast, PU.1 is not expressed in fibroblasts of healthy individuals even not after stimulation with TGF-β **(****Fig. 1C****)** suggesting the hypothesis of PU.1 as a pathophysiologically important protein in fibrotic disorders. In dermal fibroblasts from patients with SSc PU.1 is upregulated in a SMAD-dependent manner **(****Fig. 1D****).** Moreover, PU.1 is highly upregulated in different mouse models of fibrotic diseases such as bleomycin induced skin fibrosis **(****Fig. 2A****),** Tsk-1 model of fibrosis **(****Fig. 2B****),** and sclerodermatous chronic graft versus host disease (scl cGvHD) model **(****Fig. 2C****)**. The transcription factor Spi-1/PU.1 is one of the E26-transcription-specific (Ets) family of proteins and plays a central role in the maturation, differentiation and proliferation of different cell types, intensively studied in B cells and macrophages (Carotta and Nutt Immunol Rev 238, 63-75 (2010)). The functional role of PU.1 expression in fibroblasts has not been investigated so far. However, the obtained results demonstrate not only an upregulation of PU.1 in fibrotic tissues but also pro-fibrotic effects of PU.1 *in vitro* and *in vivo.*

The compound DB1976 has recently been identified as a potent inhibitor of PU.1-binding to the DNA of PU.1 target genes (Munde et al., Biochemistry 53, 1218-1227 (2014), Gillingwater et al., Veterinary Parasitology 169, 264-272 (2010), Gillingwater et al., Antimicrobial Agents and Chemotherapy 53, 5074-5079,(2009) and Stephens et al. Nucleic Acids Research 44, 4005-4013 (2016)).

Studies in accordance with the invention show a strong anti-fibrotic effect of this compound in matrix-producing fibroblasts from different tissues (skin, lung, tumour) and in different mouse models of fibrosis.

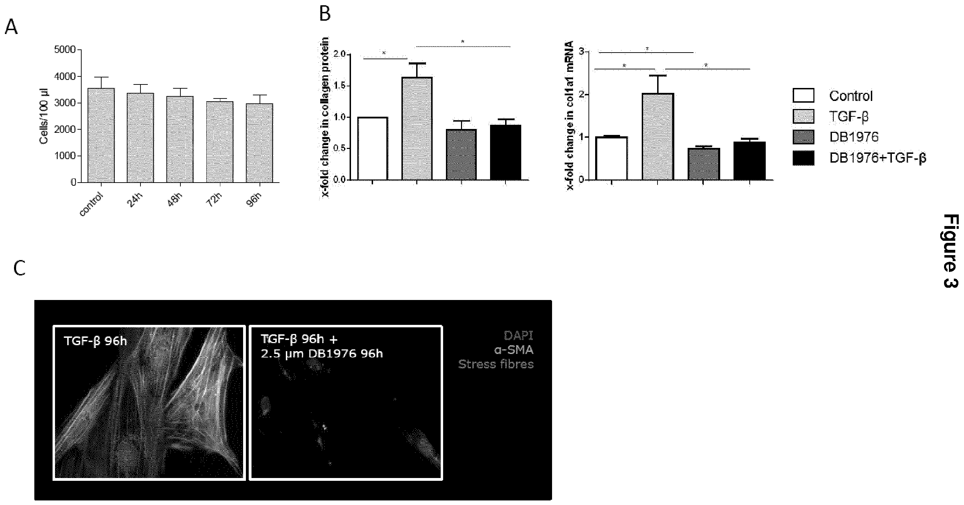

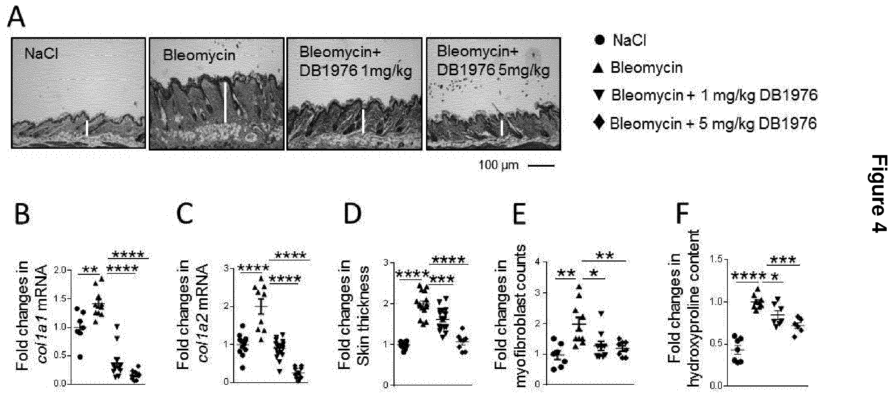

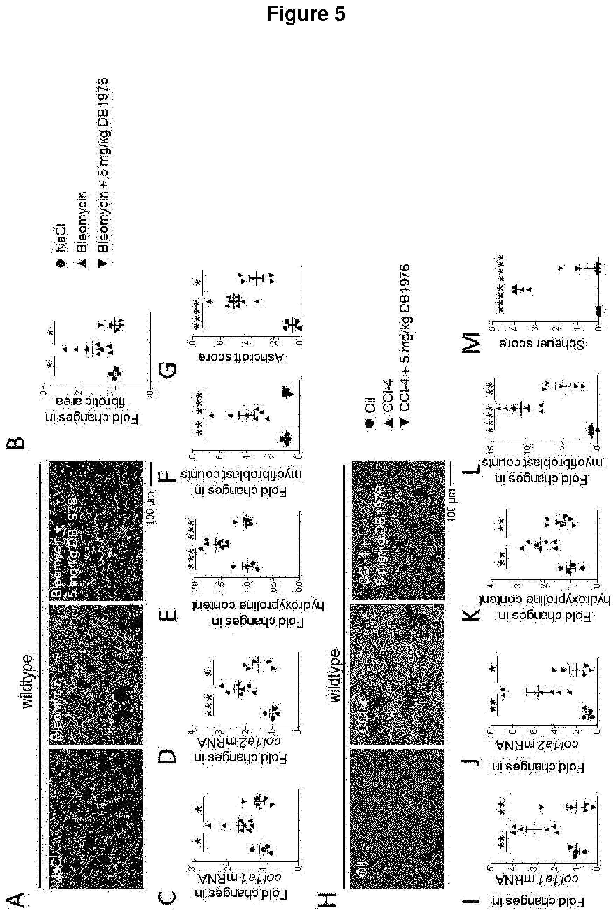

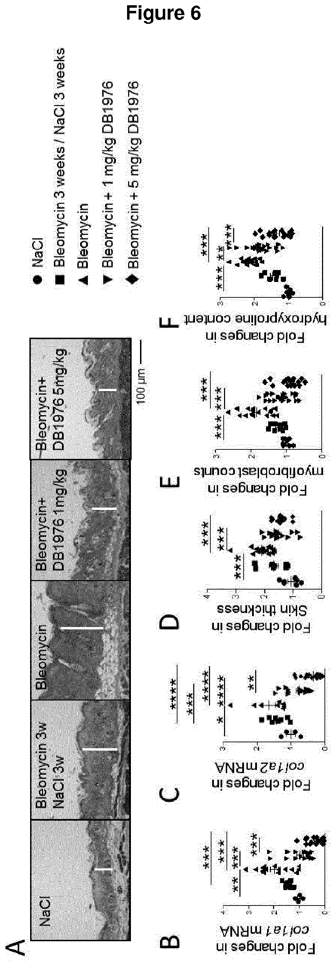

Dermal fibroblasts from patients with SSc were cultured in the presence of DB1976. Inventors did not observe toxic effects assessed in formazan toxicity assays **(****Fig. 3A****).** In the presence of DB1976 inventors found a strong reduction of collagen release from fibroblasts after stimulation with TGF-β as compared to fibroblasts which were stimulated with TGF-β alone **(****Fig. 3B****).** Moreover, in the presence of DB1976 differentiation of fibroblasts into matrix-producing myofibroblasts is inhibited **(****Fig. 3C****).** Bleomycin induced skin fibrosis was induced by local injections of bleomycin in C57BI/6 mice (10 weeks of age, mixed genders) for four weeks. Subcutaneous injections of 0.9% NaCl served as a control. DB1976 was administered into tail vein every second day in different concentrations (1 mg/kg body weight; 5 mg/kg body weight) after first application of bleomycin. Treatment with DB1976 significantly decreased skin thickening, collagen release assessed by col1a2 mRNA expression and hydroxyproline content of the skin, and myofibroblast differentiation in a concentration dependent manner **(****Fig. 4A-F****).** Bleomycin-induced pulmonary fibrosis was induced by a single intratracheal application of bleomycin in C57BI/6 mice (12 weeks of age, males) using a high pressure syringe (Penn-Century, Wyndmoor, PA, USA). Instillation of equal volumes of 0.9% NaCl served as a control. Therapeutic application of DB1976 ameliorated pulmonary fibrosis demonstrated by a decreased volume of fibrotic area, decreased collagen expression (mRNA and hydroxyproline content), reduced Ashcroft score, and a decreased myofibroblast count compared to mice that received bleomycin without DB1976 **(****Fig. 5A-G****).** Carbon tetrachloride CCl₄-induced hepatic fibrosis was induced by intraperitoneal (i.p.) injections of CCl₄ in C57BI/6 mice (14 weeks of age, mixed genders) (Palumbo-Zerr et al. Nature Medicine 21, 150-158 (2015)) twice weekly. Treatment with DB1976 led to significant less fibrotic tissue remodeling of the liver, less collagen production and less myofibroblast differentiation **(****Fig. 5H-M****).** DB1976 was also effective when initiated after fibrosis had already become manifest. In those experiments, mice were pre-challenged with bleomycin for 3 weeks to induce robust skin fibrosis. After 3 weeks, treatment with DB1976 was initiated, while injections with bleomycin were continued. After a total of 6 weeks of bleomycin and 3 weeks of treatment with DB1976, the extent of fibrosis was significantly reduced in mice treated with DB1976 compared to controls. Therapeutic application of DB1976 ameliorated skin thickness, collagen content and myofibroblast counts in the skin **(****Fig. 6A-F****).** Treatment with DB1976 was well-tolerated by mice, and there was no clinical evidence of toxicity. Inventors did not detect any evidence of apoptosis induced by DB1976 in staining for cleaved caspase-3 or by TUNEL assays.

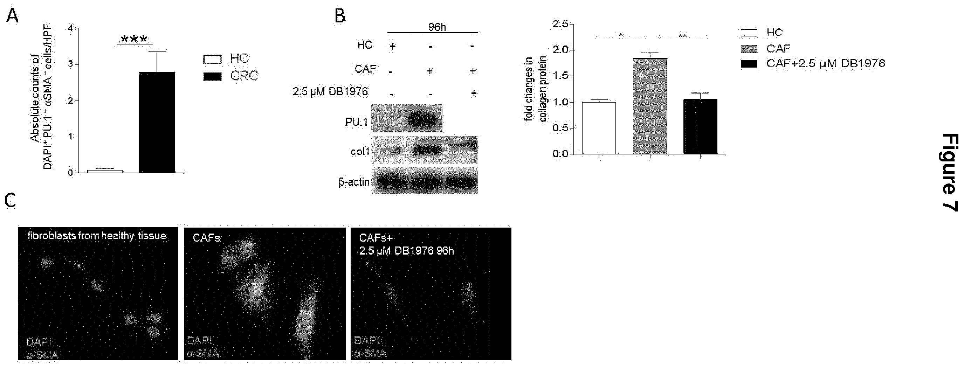

Tumors can be seen as wounds that do not heal (Kalluri and Zeisberg, Nature Reviews, Cancer 6, 392-401 (2006)). Cancer associated fibroblasts (CAFs) are associated with cancer cells at all stages of cancer progression, and their structural and functional contributions to this process are well known. This implies that CAFs have a prominent role in the progression, growth and spread of cancers and represent an important target for cancer therapies. Inventors found high upregulation of PU.1 in CAFs of different tumors **(****Fig. 7A****)** (colon cancer, pancreatic cancer) whereas fibroblasts in healthy tissue of the same patients did not express PU.1 **(****Fig. 7B****).** CAFs activated by TGF-β and cultured in the presence of DB1976 for 96h showed a significant reduction of collagen production **(****Fig. 7C****),** alpha smooth muscle actin (αSMA), and stress fiber expression compared to CAFs cultured without DB1976.

This experiment shows that DB1976 inhibits the function of CAFs to release matrix proteins that are important for angiogenic recruitment of endothelial cells and pericytes and tumor progression. DB1976 that blocks binding of PU.1 to target genes might be a new strategy for treatment of different fibrotic diseases including systemic sclerosis (*limited cutaneous scleroderma* and *diffuse cutaneous scleroderma*)*,* pulmonary fibrosis, hepatic cirrhosis, renal fibrosis, chronic graft-versus-host disease, Crohn's disease, arthrofibrosis, myelofibrosis, Dupuytren's disease and nephrogenic systemic fibrosis, breast cancer, pancreatic cancer and colon cancer.

The present invention thus provides a pharmaceutical composition comprising a PU.1 inhibitor of formula I for use in the prophylaxis or treatment of a disease associated with fibrosis.

Moreover, the present invention provides a novel pharmaceutical composition comprising a therapeutically effective amount of a compound of formula (I) 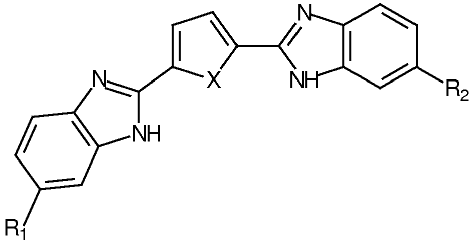
wherein X is Se, O, Te, S or NH,
and R₁ and R₂ are the same or different and are selected from 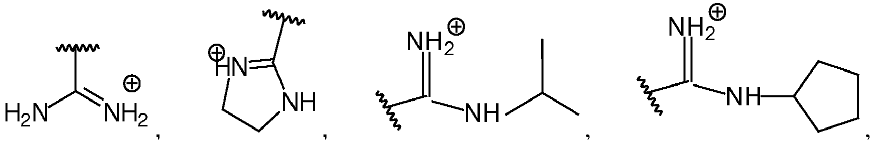 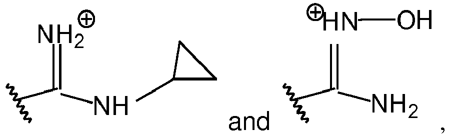
or a pharmaceutically acceptable salt thereof;
optionally comprising a carrier and one or more pharmaceutically acceptable excipients, for use in the prophylaxis or treatment of a disease associated with fibrosis.

In a preferred embodiment, the compound of general formula (I) is a compound 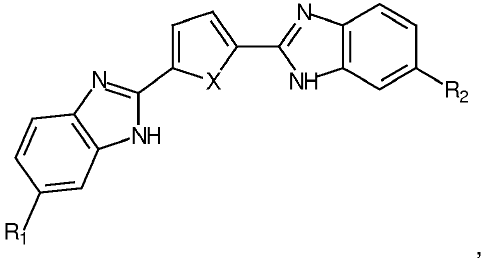 wherein X is O or Se and R₁ and R₂ are the same or different and are selected from

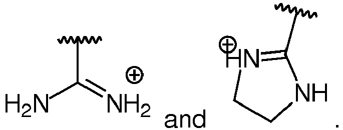

In any of the above embodiments, pharmaceutical compositions comprising a compound of general formula (I), wherein X is Se are preferred.

In any of the above embodiments, pharmaceutical compositions comprising a compound of general formula (I), wherein R₁ and R₂ are the same or different and are selected from 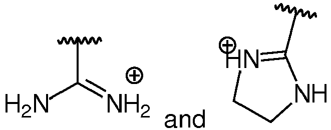 are preferred.

In any of the above embodiments, pharmaceutical compositions comprising a compound of general formula (I), wherein R₁ and R₂ are the same are preferred.

In a further preferred embodiment, the compound of formula (I) is a compound wherein X is Se and R₁ and R₂ are both

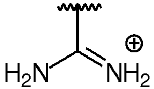

### (Compound DB1976).

In yet a further preferred embodiment, the compound of formula (I) is a compound wherein X is Se and R₁ and R₂ are both

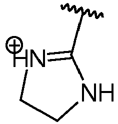

### (Compound DB1977)

In a further embodiment, the present invention provides compositions according to the present invention which may be used in the prophylaxis and/or treatment of a disease associated with fibrosis or dysregulated fibro-proliferative responses.

In a preferred embodiment of the invention, the disease is selected from a non-malignant fibrotic disease (i.e. non-cancer disease) and a malignant fibrotic disease (i.e. tumour disease), whereby the disease associated with fibrosis or dysregulated fibro-proliferative responses is preferably a non-malignant fibrotic disease.

In accordance with the present invention, the non-malignant fibrotic disease may be selected from systemic sclerosis (limited cutaneous scleroderma and diffuse cutaneous scleroderma), pulmonary fibrosis, hepatic cirrhosis, non-alcoholic steatohepatitis (NASH), alcoholic steatohepatitis, non-alcoholic fatty liver disease (NAFLD), renal fibrosis, tubulointerstitial fibrosis, glomerulosclerosis, vascular sclerosis, chronic graft-versus host disease, Crohn's disease, colitis ulcerosa, collagenous colitis, arthrofibrosis, myelofibrosis, Dupuytren's disease, nephrogenic systemic fibrosis, atherosclerosis, restenosis, cardiac fibrosis (including myocardial and endocardial fibrosis), pulmonary hypertension, muscle fibrosis, osteoporosis, excessive scarring, keloid and hypertrophic scar development, morphea, macular degeneration, retinal and vitreal retinopathy and ocular scarring.

It is further preferred that the non-malignant disease is selected from systemic sclerosis (including limited cutaneous scleroderma and diffuse cutaneous scleroderma), pulmonary fibrosis, hepatic cirrhosis, renal fibrosis, chronic graft-versus-host disease, Crohn's disease, ulcerative colitis, arthrofibrosis, myelofibrosis, Dupuytren's disease, myopathies and nephrogenic systemic fibrosis.

In accordance with the present invention, malignant fibrotic disease may be selected from mamma carcinoma, endometrial adenocarcinoma, ovarian serous tumor, lung adenocarcinoma, lung squamous cell tumor, colorectal tumor, pancreatic tumor, non-small cell lung cancer (NSCLC), squamous cell carcinoma (such as squamous cell carcinoma of the lung, cervix, skin and the gastrointestinal tract, e.g., esophagus), basal cell carcinoma, gastric carcinoma, intestinal type, gastric carcinoma, diffuse type (mucinous), adenocarcinoma (colon), hepatocellular carcinoma, renal cell carcinoma, endometrioid carcinoma of endometrium, invasive carcinoma of the breast, carcinoma metastasis (lymph nodes, bones or other organs), prostate cancer and thyroid cancer.

In yet a further preferred embodiment, the malignant disease is a tumour selected from mamma carcinoma, endometrial adenocarcinoma, ovarian serous tumor, lung adenocarcinoma, lung squamous cell tumor, colorectal tumor and pancreatic tumor.

According to another embodiment, the pharmaceutical composition according to the present invention may be administered in combination with at least one compound selected from the group of an anti proliferative agent or an anti cancer agent and/or therapy, such as radiotherapy. Possible active compounds/classes of active compounds for combination may be alkylating agents, antimetabolites, anti-microtubule agents, topoisomerase inhibitors, cytotoxic antibiotics or antibody-drug conjugates.

Alkylating agents to be used in accordance with the present invention may be selected from active agents such as cyclophosphamid, chlormethine, uramustine, melphalan, chlorambucil, ifosfamide, bendamustine, carmustine, lomustine, dacarbazine, streptozocin, busulfan, cisplatin, carboplatin, nedaplatin, oxaliplatin, satraplatin, triplatin tetranitrate, procarbazine and altretamine, but are not limited to said active agents.

Antimetabolites to be used in accordance with the present invention comprise, but are not limited to, folic acid analogues, purine analogues and pyrimidine analogues such as 5-fluorouracil, mercaptopurine, gemcitabine, methotrexat, capecitabine, thioguanine, pemetrexed, fludarabine, clofarabine and cladribine.

According to the present invention, antimicrotubule agents may, e.g., be selected from, but are not limited to, vinca alkaloids such as vincristine, vinblastine and vinorelbine, taxanes such as paclitaxel, nab-paclitaxel, docetaxel and cabazitaxel, and Epothilone B analogues **such as** ixabepilone.

Topoisomerase inhibitors may be selected from, however, are not limited to irinotecan, topotecan, camphotericin, lamellarinD, etoposide, teniposide, doxorubicin, daunorubicin, mitoxantone, amsacrine, ellipticines, aurintricarbocyclic acid and HU-331.

*Cytotoxic antibiotics* may be selected from, but are not limited to, active agents such as anthracyclines such as doxorubincin, daunorubicin, epirubicin, idarubicin, bleomycine, mitomycine C, mitoxantrone and actinomycin.

Antibody-drug conjugates (ADCs) in accordance with the above such as described in Teicher BA, Chari RV (Oct 2011). "Antibody conjugate therapeutics: challenges and potential". Clinical Cancer Research. 17 (20): 6389-97. PMID 22003066. doi:10.1158/1078-0432.CCR-11-1417*.*

The present invention also pertains to the pharmaceutical compositions provided herein, wherein the corresponding compound or pharmaceutical composition is to be administered by any one of: an oral route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; or ophthalmic route, including by intravitreal, or intracameral route. Particularly preferred routes for administration are parenteral administration like, e.g., intravenous and intraperitoneal (during hyperthermic chemoperfusion), local administration such as topical administration or administration by inhalation or insufflation, and oral administration, whereby subcutaneous administration is particularly preferred.

### Figure legends

**Figure 1****: PU.1 expression in fibrotic skin. (A)** Quantification of PU.1-expressing fibroblasts in healthy skin compared to fibrotic skin. Cells are counted per high power field (HPF). **(B)** Confocal microscopy of fibrotic skin; fibroblasts are identified as prolyl-4-hydroxylase positive cells and co-stained with PU.1 and DAPI. Representative image out of 5 independent experiments is shown. **(C)** PU.1 expression in fibroblasts from healthy skin compared to fibroblasts from fibrotic skin after stimulation with TGF-b for 1-72h assessed by western blot analysis; β-actin expression served as control. **(D)** SMAD dependent expression of PU.1 assessed by western blot analysis; fibroblasts are transfected with SMAD3 siRNA or control siRNA before stimulation with TGF-β. Total Smad3, phosphorylated Smad3 (pSmad3), PU.1, and β-actin are assessed. Representative western blots of 5 independent experiments are shown. **p<0.01; ***p<0.001 determined by *Mann-Whitney U*-test for non-parametric data.
**Figure 2****: PU.1 expression in different mouse models of fibrotic diseases. (A)** Bleomycin induced skin fibrosis, **(B)** Tight-skin 1 (Tsk-1) model of fibrosis, **(C)** sclerodermatous chronic Graft versus Host Disease (scl cGvHD) mouse model of fibrosis; mice transplanted with bone marrow from syngenic mice (syn BMT) that do not develop fibrotic skin are compared to mice transplanted with bone marrow from allogenic mice (allo BMT) developing skin fibrosis. PU.1 and col1a2 mRNA expression levels are shown relative to control. Quantification of PU.1-expressing, vimentin positive fibroblasts per high power field (HPF). Data are shown as mean ± SEM. *p<0.05; **p<0.01; ***p<0.001 determined by *Mann-Whitney U test* for non-parametric data.
**Figure 3****: DB1976 shows anti-fibrotic effects** *in vitro.* **(A)** Formazan toxicity assay of fibroblasts incubated with different concentrations of DB1976 as indicated; **(B)** human diseased dermal fibroblasts untreated or stimulated with either TGF-β alone or TGF-β plus DB1976; collagen protein level and mRNA level of col1a1 relative to control. Data are shown as mean ± SEM. **(C)** Immune fluorescence microscopy of cultured fibroblasts as indicated; cells are stained with DAPI, alpha smooth muscle actin (αSMA) and stress fibers. Representative image of 5 independent experiments are shown. *p<0.05 determined by *Student's t* test according pretest for normality (D'Agostino-Pearson normality test).
**Figure 4****: Model of bleomycin induced skin fibrosis. (A)** Representative images of trichrome staining of skin tissue from control mice (Wild-type (WT) treated with sodium chloride; n≥7), bleomycin treated mice (n=10 ) and mice treated with bleomycin and different concentrations of DB1976 (n≥8each); **(B;C)** mRNA collagen1a1 or collagen1a2 expression; **(D)** dermal thickness; **(E)** quantification of α-smooth muscle actin-positive myofibroblasts; **(F)** hydroxyproline content. Results are demonstrated relative to control and as mean ± SEM. *p<0.05; **p<0.01; ***p<0.001 determined by determined by 1-Way ANOVA.
**Figure 5****: Model of bleomycin induced lung and CCI-4 induced liver fibrosis. (A)** Representative images of sirius red staining of lung tissue from control mice (Wild-type (WT) treated with sodium chloride; n=4), bleomycin treated mice (n≥7) and mice treated with bleomycin and DB1976 (n=≥5); **(B)** quantification of the fibrotic area; **(C, D)** mRNA expression level of col1a1 and col1a2; **(E)** hydroxyproline content. **(F)** quantification of α-smooth muscle actin-positive myofibroblasts **(G)** Ashcroft score; **(H)** Representative images of sirius red staining of liver tissue from control mice (Wild-type (WT) treated with sunflower oil; n=4), carbon tetrachloride (CCl-4) treated mice (n=6) and mice treated with CCI-4 and DB1976 (n≥5); **(I,J)** mRNA expression level of col1a1 and col1a2; **(K)** hydroxyproline content. **(L)** quantification of α-smooth muscle actin-positive myofibroblasts **(M)** Scheuer score; Results are demonstrated relative to control and as mean ± SEM. *p<0.05; **p<0.01; ***p<0.001 determined by 1-Way ANOVA.
**Figure 6****: Regression of pre-established bleomycin induced skin fibrosis by treatment with DB1976.** Mice were pre-challenged with bleomycin for 3 weeks to induce robust skin fibrosis. After 3 weeks, treatment with DB1976 was initiated, while injections with bleomycin were continued. After a total of 6 weeks of bleomycin and 3 weeks of treatment with DB1976, the extent of fibrosis was assessed. **(A)** Representative images of trichrome staining of skin tissue from control mice (Wild-type (WT) treated with sodium chloride; n≥4), 3 weeks bleomycin and afterwards 3 weeks sodium chloride treated mice (n≥8), bleomycin treated mice (n≥8) and mice treated with bleomycin and different concentrations of DB1976 (n≥3each); **(B;C)** mRNA expression level of col1a1 and col1a2; **(D)** dermal thickness; **(E)** quantification of α-smooth muscle actin-positive myofibroblasts; **(F)** hydroxyproline content. Results are demonstrated relative to control and as mean ± SEM. *p<0.05; **p<0.01; ***p<0.001 determined by 1-Way ANOVA
**Figure 7****: DB1976 inhibits collagen production of cancer associated fibroblasts (CAFs). (A)** Quantification of PU.1-expressing fibroblasts in healthy colon tissue compared to tumor tissue (colorectal carcinoma). Cells are counted per high power field (HPF). (B) collagen production of CAFs assessed by western blot analysis; PU.1, collagen and β-actin expression in response to stimulation w/o TGF-β in the presence and absence of DB1976; representative blots of 6 independent experiments are shown. **(B)** Quantification of collagen production of CAFs cultured w/o DB1976 relative to collagen production of fibroblasts isolated from healthy tissue (HC); results are demonstrated as mean ± SEM. **(C)** Immune fluorescence microscopy of fibroblasts from healthy tissue (HC) and CAFs cultured w/o DB1976; DAPI and alpha smooth muscle actin (aSMA) are stained. Representative images of 5 independent experiments are shown. *p<0.05; **p<0.01 determined by *Mann-Whitney U*-test for non-parametric data.

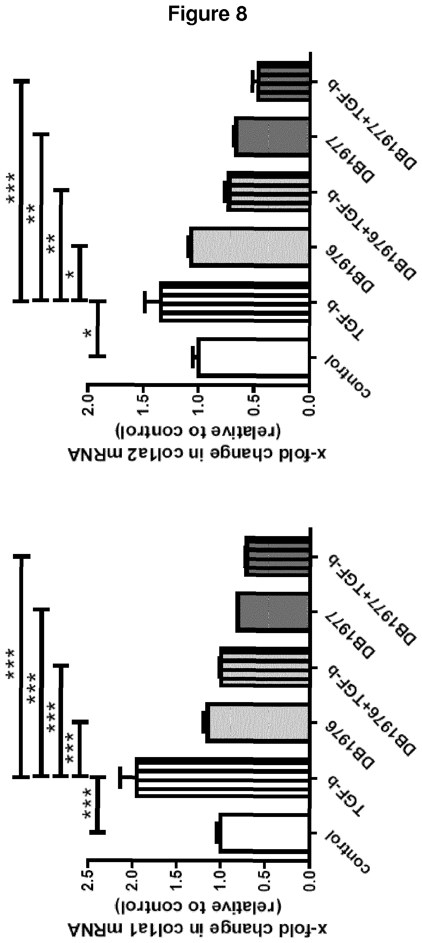

Also compound DB1977 exhibited strong reduction of collagen release from fibroblasts after stimulation with TGF-β as compared to fibroblasts which were stimulated with TGF-β alone (Fig. 8; assay as illustrated in Fig. 3).

### Definitions

The following definitions apply throughout the present specification, unless specifically indicated otherwise.

As used herein, the term" inhibiting" or "inhibition" refers to the ability of a compound to down regulate, decrease, reduce, suppress, inactivate or inhibit at least partially the activity of an enzyme or the expression of an enzyme or protein.

As used herein, the terms "optional", "optionally" and "may" denote that the indicated feature may be present but can also be absent. Whenever the term "optional", "optionally" or "may" is used, the present invention specifically relates to both possibilities, i.e. that the corresponding feature is present or, alternatively, that the corresponding feature is absent.

As used herein, unless explicitly indicated otherwise or contradicted by context, the terms "a", "an" and "the" are used interchangeably with "one or more" and "at least one".

Pharmaceutically acceptable salts of the compounds of the invention of formula (I) can be formed with numerous organic and inorganic acids. Exemplary acid addition salts including acetate, adipate, alginate, ascorbate, aspartate, benzoate, benzenesulfonate, bisulphate, borate, butyrate, citrate, camphorate, camphersulfonate, cyclopentanepropionate, digluconate, dodecyl sulphate, ethane sulfonate, fumarate, glucoheptanoate, glycerophosphate, hemisulphate, heptanoate, hexanoate, hydrochloride, hydrobromide, hydroiodide, 2-hydroxyethane sulfonate, lactate, maleate, methane sulfonate, 2-naphthalene sulfonate, nicotinate, nitrate, oxalate, pamoate, pectinate, persulphate, 3-phenyl sulfonate, 3-phenylpropionate, phosphate, picrate, pivalate, propionate, salicylate, succinate, sulphate, sulfonate, tartrate, thiocyanate, toluene sulfonate such as tosylate, undecanoate, or the like.

Basic nitrogen-containing moieties can be quarternized with such agents as lower alkyl halides, such as methyl, ethyl, propyl, and butyl chloride, bromide and iodide; dialkyl sulfates like dimethyl, diethyl, dibutyl, and diamyl sulfates, long-chain alkyl halides such as decyl, lauryl, myristyl and stearyl chloride, bromide and iodide, or aralkyl halides like benzyl and phenethyl bromides, or others. Water soluble or dispersible products are thereby obtained.

If the compound of formula (I) is in the form of a pharmaceutically acceptable salt, it is preferably in the form of a hydrochloride salt.

Pharmaceutically acceptable salts of the compounds of the present invention may be synthesised from the parent compounds which contain a basic moiety by conventional chemical methods. Generally, such salts can be prepared by reacting the free base form of these compounds with a stochiometric amount of the appropriate acid in water, an organic solvent or a mixture thereof.

Moreover, the scope of the invention embraces the compounds of formula (I) in any solvated form, including, e.g., solvates with water (i.e. as a hydrate) or solvates with organic solvents such as, e.g., methanol, ethanol or acetonitrile (i.e. as a methanolate, ethanolate or acetonitrilate), or in any crystalline form (i.e. as any polymorph), or in amorphous form. It is to be understood that such solvates of the compounds of the formula (I) also include solvates of pharmaceutically acceptable salts of the compounds of the formula (I).

The term "pharmaceutically acceptable" is used herein to refer to those compounds, materials, compositions, and/or dosage forms which are within the scope of the sound medical judgment suitable for use in contact with the tissues of human and animals without excessive toxicity, irritation, allergic response, or other problem or complication commensurate with a reasonable risk/benefit ratio.

The term "therapeutically effective amount" of a compound of the present invention refers to an amount sufficient to effect desired clinical results (i.e. achieve therapeutic efficacy).

The term "treatment" or "therapy" of a disorder or disease as used herein (e.g., "treatment" of cancer) is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e. diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia,* comprise curative treatment (preferably leading to a complete response and eventually to healing of the disorder or disease) and palliative treatment (including symptomatic relief).

The term "treatment" as used in accordance with the present invention is meant to encompass also prevention/prophylaxis, unless indicated otherwise.

The terms "prevention" and "prophylaxis "of a disorder or disease as used herein (e.g., "prevention/prophylaxis" of cancer) are also well known in the art. They are used interchangeably throughout the specification. For example, a patient/subject suspected of being prone to suffer from a disorder or disease may particularly benefit from a prevention/prophylaxis of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the terms "prevention" and "prophylaxis" comprise the use of a compound of the present invention before any clinical and/or pathological symptoms are diagnosed or determined or can be diagnosed or determined by the attending physician.

It is to be understood that the present invention specifically relates to each and every combination of features and embodiments described herein, including any combination of general and/or preferred features/embodiments.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

### Dosage and formulation

A person of skill in the art such as a physician will be able to determine the actual dosage, which will be most suitable for an individual subject. The specific dose and frequency of dosing for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

For the purpose of the present invention, a therapeutically effective dosage will generally be from about 50 to about 500 mg/m²/day, which may be administered in one or multiple doses.

It will be appreciated, however, that specific dose levels of the compounds of the invention for any particular patient will depend on a variety of factors such as age, sex, body weight, general health condition, diet, individual response of the patient to be treated at the time of administration, severity of the disease to be treated, the activity of particular compound applied, dosage form, mode of application and concomitant medication. The therapeutically effective amount for a given situation will readily be determined by routine experimentation and is within the skills and judgement of the ordinary clinician or physician.

The unit dose may be administered, e.g., daily, weekly or once every two weeks. It will be appreciated that it may be necessary to make routine variations to the dosage depending on the age and weight of the patient/subject as well as the severity of the condition to be treated. The precise dose and also the route of administration will ultimately be at the discretion of the attendant physician.

The compositions of the present invention may be administered by oral route; parenteral route using injection techniques or infusion techniques, including by subcutaneous, intradermal, transdermal, transmucosal, subdural, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; local or topical, e.g., via iontophoresis, sublingual, by pulmonary route, including by inhalation or insufflation therapy; gastrointestinal route; intrauterine route; intraocular route; subcutaneous route; or ophthalmic route, including by intravitreal, or intracameral route or by rectal route. Particularly preferred routes of administration are parenteral administration like, e.g., intravenous and intraperitoneal (during hyperthermic chemoperfusion), local administration, e.g., topical or pulmonal administration, and oral administration, whereby subcutaneous administration is particularly preferred.

The compounds provided by the present invention may be administered as compounds *per se* or may be formulated as pharmaceutical compositions. The pharmaceutical compositions may optionally comprise one or more pharmaceutically acceptable excipients, such as carriers, diluents, fillers, disintegrants, lubricating agents, binders, colourants, pigments, stabilisers, preservatives, antioxidants, and/or solubility enhancers, as described in more detail below.

The pharmaceutical compositions comprising a compound of formula (I) may thus be administered to a subject by any convenient route of administration, whether systemically/peripherally or at the site of desired action, including but not limited to one or more of: oral (e.g., as a tablet, capsule, or as an ingestible solution), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal (in particular in form of hyperthermic chemoperfusion), intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, or ophthalmic (including intravitreal or intracameral) administration.

The pharmaceutical compositions can be formulated as dosage forms for oral, parenteral, such as intramuscular, intravenous, subcutaneous, intradermal, intraarterial, or intraperitoneal (especially in form of hyperthermic chemoperfusion) administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders, and granules for reconstitution.

Oral and parenteral, in particular intravenous administration is preferred, whereby oral administration is particularly preferred.

Dosage forms for oral administration include tablets, capsules, lozenges, pills, wafers, granules, oral liquids such as syrups, suspensions, solutions, emulsions, powder for reconstitution.

Dosage forms for parenteral administration include aqueous or oleaginous solutions or emulsions for infusion, aqueous or oleaginous solutions, suspensions or emulsions for injection pre-filled syringes, and/or powders for reconstitution.

Dosage forms for local/topical administration comprise insufflations, aerosols, metered aerosols, transdermal therapeutic systems, medicated patches, rectal suppositories, and/or ovula.

Dosage forms for oral administration selected from tablets, capsules are preferred.

The amount of the compound of the present invention that may be combined with the excipients to formulate a single dosage form will vary upon the patient treated and the particular mode of administration.

The compounds of formula (I) may be formulated into pharmaceutical compositions using one or more conventional pharmaceutically acceptable excipient(s) commonly used in formulation technology, e.g., such as *inter alia* referred to in Fiedler's "Lexikon der Hilfstoffe" 5th Edition, Editio Cantor Verlag Aulendorf 2002, "The Handbook of Pharmaceutical Excipients", 4th Edition, American Pharmaceuticals Association, 2003, and may be selected from carriers, diluents or fillers, binding agents, disintegrants, lubricants, glidants, stabilising agents, surfactants, film-formers, softeners, wetting agents, sweeteners, pigments/colouring agents, antioxidants, preservatives and the like. Suitable carriers, binding agents, disintegrants, lubricants and glidants can, e.g., be the ones described in more detail here above as pharmaceutically acceptable auxiliary agents.

Excipients that may be used in the formulation of the pharmaceutical compositions of the present invention comprise carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate; binders, adjuvants, solubilisers, thickening agents, stabilisers, disintegrants, glidants, lubricating agents, buffering agents, emulsifiers, wetting agents, suspending agents, sweetening agents, colourants, flavours, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-β-cyclodextrin, polyvinylpyrrolidone, low melting waxes, ion exchange resins.

Other suitable pharmaceutically acceptable excipients are described in Remington's Pharmaceutical Sciences, 15th Ed., Mack Publishing Co., New Jersey (1991).

The pharmaceutical compositions can be formulated by techniques known to the person skilled in the art, such as the techniques published in "Remington: The Science and Practice of Pharmacy", Pharmaceutical Press, 22nd edition, "Pharmazeutische Technologie", 11th Edition Deutscher Apotheker Verlag 2010, or "Pharmazeutische Technologie", 9th Edition Wissenschaftliche Verlagsgesellschaft Stuttgart, 2012.

For parenteral administration, the compounds are best used in the form of a sterile aqueous solution, which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavouring or colouring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

Lists of further suitable excipients may also be found in textbooks such as Remington's Pharmaceutical Sciences, 18th Ed. (Alfonso R. Gennaro, ed.; Mack Publishing Company, Easton, PA, 1990); Remington: the Science and Practice of Pharmacy 19th Ed. (Lippincott, Williams & Wilkins, 1995); Handbook of Pharmaceutical Excipients, 3rd Ed. (Arthur H. Kibbe, ed.; Amer. Pharmaceutical Assoc, 1999); the Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E. Limbird, eds.; McGraw Hill, 1992).

Said pharmaceutical compositions may also be administered by sustained-release systems. Suitable examples of sustained-release compositions include semi-permeable polymer matrices in the form of shaped articles, e.g., films, or microcapsules. Sustained-release matrices include, e.g., polylactides (see, e.g., US 3,773,919), copolymers of L-glutamic acid and gamma-ethyl-L-glutamate (Sidman, U. et al., Biopolymers 22:547-556 (1983)), poly(2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133 988). Sustained-release pharmaceutical compositions also include liposomally entrapped compounds. Liposomes containing a compound of the present invention can be prepared by methods known in the art, such as, e.g., the methods described in any one of: DE 32 18 121; Epstein et al., Proc. Natl. Acad. Sci. (USA) 82:3688-3692 (1985); Hwang et al., Proc. Natl. Acad. Sci. (USA) 77:4030-4034 (1980); EP 0 052 322; EP 0 036 676; EP 0 880 46; EP 0 143 949; EP 0 142 641; JP 83-118008; US 4,485,045; US 4,544,545; and EP 0 102 324.

Said pharmaceutical compositions may also be administered by the pulmonary route or the ocular route. For ophthalmic use, they can be formulated as micronised suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

The pharmaceutical composition comprising the compound of formula (I) can be administered in monotherapy (e.g., without concomitant administration of any further therapeutic agents or, in particular, without concomitant administration of any further antiproliferative agents or anticancer drugs). However, the pharmaceutical composition comprising the compound of formula (I) can also be administered in combination with at least one further therapeutic agent (i.e. one or more further therapeutic agents) and/or radiotherapy.

If the composition comprising the compound of formula (I) is used in combination with at least a second therapeutic agent active against the same disease or condition (e.g., a further anticancer drug), the dose of each compound may differ from that when the corresponding compound is used alone, in particular, a lower dose of each compound may be used. The combination of the composition comprising the compound of formula (I) with one or more further therapeutic agents may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in one or more different (separate) pharmaceutical formulations.

Preferably, the one or more further therapeutic agents to be administered in combination with a compound of the present invention are antiproliferative agents or anticancer drugs such as alkylating agents, antimetabolites, anti-microtubule agents, topoisomerase inhibitors, cytotoxic antibiotics, and antibody-drug conjugates as disclosed above.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (i.e. the compound of formula (I) or a pharmaceutically acceptable salt or solvate thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation.

The composition comprising the compound of formula (I) can also be administered in combination with physical therapy, such as radiotherapy. Radiotherapy may commence before, after, or simultaneously with administration of the compounds of the invention. For example, radiotherapy may commence 1-10 minutes, 1-10 hours or 24-72 hours after administration of the compounds. Yet, these time frames are not to be construed as limiting. The subject is exposed to radiation, preferably gamma radiation, whereby the radiation may be provided in a single dose or in multiple doses that are administered over several hours, days and/or weeks. Gamma radiation may be delivered according to standard radio therapeutic protocols using standard dosages and regimens.

The subject or patient to be treated in accordance with the present invention may be an animal (e.g., a non-human animal), or a human. Preferably, the subject/patient is a mammal. More preferably, the subject/patient is a human.

## Claims

1. A pharmaceutical composition comprising a compound of the general formula (I) 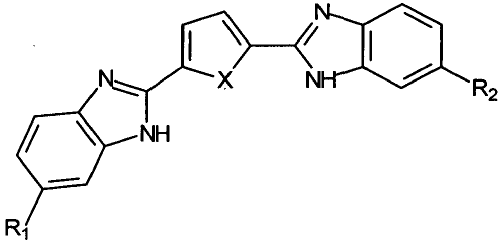
wherein X is Se, O, Te, S or NH,
and R₁ and R₂ are the same or different and are selected from 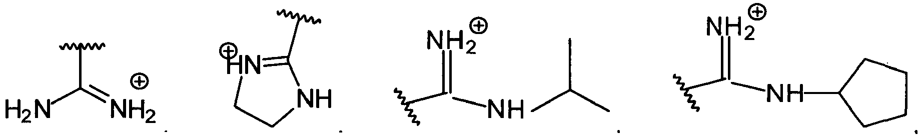 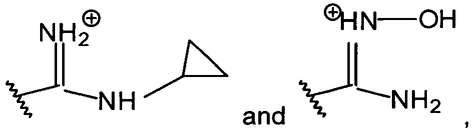
or a pharmaceutically acceptable salt thereof;
optionally comprising a carrier and one or more pharmaceutically acceptable excipients, for use in the prophylaxis or treatment of a disease associated with fibrosis, wherein the disease is non-malignant or malignant and wherein the non-malignant disease is selected from systemic sclerosis *(limited cutaneous scleroderma* and *diffuse cutaneous scleroderma),* pulmonary fibrosis, hepatic cirrhosis, non-alcoholic steatohepatitis (NASH), alcoholic steatohepatitis, non-alcoholic fatty liver disease (NAFLD), renal fibrosis, tubulointerstitial fibrosis, glomerulosclerosis, vascular sclerosis, chronic graft-versus host disease, Crohn's disease, colitis ulcerosa, collagenous colitis, arthrofibrosis, myelofibrosis, Dupuytren's disease, nephrogenic systemic fibrosis, atherosclerosis, restenosis, cardiac fibrosis, pulmonary hypertension, muscle fibrosis, osteoporosis, excessive scarring, keloid and hypertrophic scar development, morphea, macular degeneration, retinal and vitreal retinopathy and ocular scarring and the malignant disease is selected from mamma carcinoma, endometrial adenocarcinoma, ovarian serous tumor, lung adenocarcinoma, lung squamous cell tumor, colorectal tumor, pancreatic tumor, non-small cell lung cancer (NSCLC), squamous cell carcinoma, basal cell carcinoma, gastric carcinoma, intestinal type, gastric carcinoma, diffuse type (mucinous), adenocarcinoma (colon), hepatocellular carcinoma, renal cell carcinoma, endometrioid carcinoma of endometrium, invasive carcinoma of the breast, carcinoma metastasis, prostate cancer and thyroid cancer.

2. Pharmaceutical composition for use according to claim 1, comprising a compound of the general formula (I) 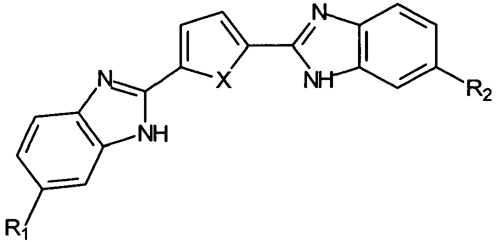
wherein X is Se or O,
and R₁ and R₂ are the same or different and are selected from 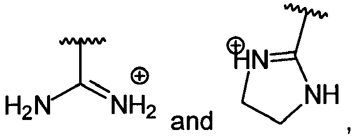
or a pharmaceutically acceptable salt thereof;
optionally comprising a carrier and one or more pharmaceutically acceptable excipients .

3. Pharmaceutical composition for use according to claim 1 or 2, wherein X is Se.

4. Pharmaceutical composition for use according to claims 1 to 3, wherein R₁ = R₂.

5. Pharmaceutical composition for use according to claims 1 to 4, wherein X is Se and R₁ and R₂ are both 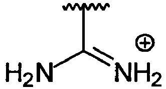 (compound DB1976).

6. Pharmaceutical composition for use according to claims 1 to 4, wherein X is Se and R₁ and R₂ are both 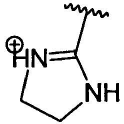 (compound DB1977).

7. Pharmaceutical composition for use according to any one of claims 1 to 6 for oral, topical or parenteral administration.

8. Pharmaceutical composition for use according to any of claims 1 to 7, wherein the disease is non-malignant.

9. Pharmaceutical composition for use according to any of claims 1 to 8, wherein the disease is selected from systemic sclerosis (limited cutaneous scleroderma and diffuse cutaneous scleroderma), pulmonary fibrosis, hepatic cirrhosis, renal fibrosis, chronic graft-versus-host disease, Crohn's disease, arthrofibrosis, myelofibrosis, Dupuytren's disease and nephrogenic systemic fibrosis.

10. Pharmaceutical composition for use according to any of claims 1 to 7, wherein the disease is selected from mamma carcinoma, endometrial adenocarcinoma, ovarian serous tumor, lung adenocarcinoma, lung squamous cell tumor, colorectal tumor and pancreatic tumor.

11. Pharmaceutical composition for use according to any of claims 1 to 9, which is to be administered in combination with at least one anti proliferative agent selected from the group of alkylating agents, antimetabolites, anti-microtubule agents, topoisomerase inhibitors, cytotoxic antibiotics, and antibody-drug conjugates.

12. Pharmaceutical composition for use according to any of claims 1 to 10, which is to be administered in combination with at least one anti cancer agent selected from the group of alkylating agents, antimetabolites, anti-microtubule agents, topoisomerase inhibitors, cytotoxic antibiotics, antibody-drug conjugates and/or radiotherapy.

## Patentansprüche

1. Ein Arzneimittel, umfassend eine Verbindung der allgemeinen Formel (I) 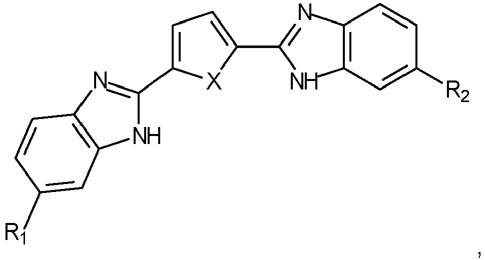
wobei X für Se, O, Te, S oder NH steht,
und R₁ und R₂ gleich oder verschieden sind und ausgewählt sind aus 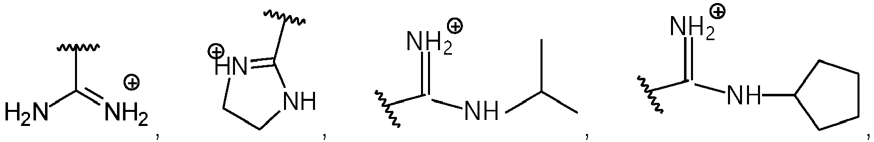 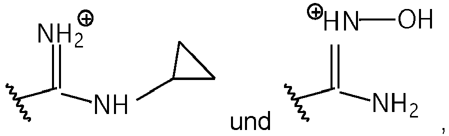
oder ein pharmazeutisch verträgliches Salz davon;
gegebenenfalls umfassend einen Träger und einen oder mehrere pharmazeutisch verträgliche Exzipienten zur Verwendung bei der Vorbeugung oder Behandlung einer Erkrankung, welche in Zusammenhang mit Fibrose steht, wobei die Erkrankung gutartig oder bösartig ist und wobei die gutartige Erkrankung ausgewählt ist aus systemischer Sklerose (*limitierte kutane Sklerodermie* und *diffuse kutane Sklerodermie*)*,* Lungenfibrose, Leberzirrhose, nichtalkoholischer Steatohepatitis (NASH), alkoholischer Steatohepatitis, nichtalkoholischer Fettlebererkrankung (NAFLD), Nierenfibrose, tubulointerstitieller Fibrose, Glomerulosklerose, vaskulärer Sklerose, chronischer Graft-versus-Host-Erkrankung, Morbus Crohn, Colitis ulcerosa, kollagener Kolitis, Arthrofibrose, Myelofibrose, Dupuytren-Erkrankung, nephrogener systemischer Fibrose, Atherosklerose, Restenose, kardialer Fibrose, pulmonaler Hypertonie, Muskelfibrose, Osteoporose, übermäßiger Narbenbildung, Keloid und hypertropher Narbenbildung, Morphea, Makuladegeneration, retinaler und vitrealer Retinopathie und okularer Narbenbildung, und die bösartige Erkrankung ausgewählt ist aus Mammakarzinom, Endometrium-Adenokarzinom, serösem Eierstocktumor, Lungen-Adenokarzinom, Lungen-Plattenepithelkarzinom, kolorektalem Tumor, Tumor der Bauchspeicheldrüse, nicht-kleinzelligem Lungenkrebs (NSCLC), Plattenepithelkarzinom, Basalzellenkarzinom, Magenkarzinom, intestinaler Typ, Magenkarzinom, diffuser Typ (muzinös), Adenokarzinom (Dickdarm), Leberzellkarzinom, Nierenzellkarzinom, Endometriumkarzinom des Endometriums, invasivem Karzinom der Brust, Karcinomametastasen, Prostatakrebs und Schilddrüsenkrebs.

2. Arzneimittel zur Verwendung gemäß Anspruch 1, umfassend eine Verbindung der allgemeinen Formel (I) 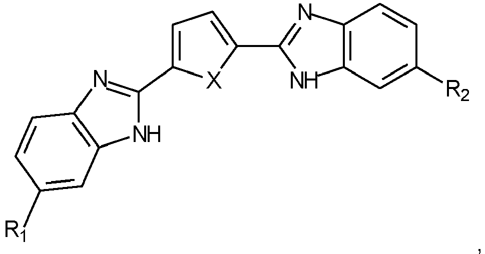
wobei X für Se oder O steht,
und R₁ und R₂ gleich oder verschieden sind und ausgewählt sind aus 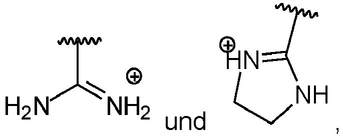
oder ein pharmazeutisch verträgliches Salz davon;
gegebenenfalls umfassend einen Träger und einen oder mehrere pharmazeutisch verträgliche Exzipienten.

3. Arzneimittel zur Verwendung gemäß Anspruch 1 oder 2, wobei X für Se steht.

4. Arzneimittel zur Verwendung gemäß den Ansprüchen 1 bis 3, wobei R₁ = R₂.

5. Arzneimittel zur Verwendung gemäß den Ansprüchen 1 bis 4, wobei X für Se steht und R₁ und R₂ beide 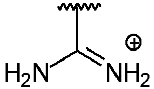 (Verbindung DB1976)
darstellen.

6. Arzneimittel zur Verwendung gemäß den Ansprüchen 1 bis 4, wobei X für Se steht und R₁ und R₂ beide 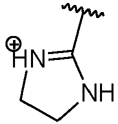 (Verbindung DB1977)
darstellen.

7. Arzneimittel zur Verwendung gemäß einem der Ansprüche 1 bis 6 zur oralen, topischen oder parenteralen Verabreichung.

8. Arzneimittel zur Verwendung gemäß einem der Ansprüche 1 bis 7, wobei die Erkrankung gutartig ist.

9. Arzneimittel zur Verwendung gemäß einem der Ansprüche 1 bis 8, wobei die Erkrankung ausgewählt ist aus systemischer Sklerose (limitierte kutane Sklerodermie und diffuse kutane Sklerodermie), Lungenfibrose, Leberzirrhose, Nierenfibrose, chronischer Graft-versus-Host-Erkrankung, Morbus Crohn, Arthrofibrose, Myelofibrose, Dupuytren-Erkrankung und nephrogener systemischer Fibrose.

10. Arzneimittel zur Verwendung gemäß einem der Ansprüche 1 bis 7, wobei die Erkrankung ausgewählt ist aus Mammakarzinom, Endometrium-Adenokarzinom, serösem Eierstocktumor, Lungen-Adenokarzinom, Lungen-Plattenepithelkarzinom, kolorektalem Tumor und Tumor der Bauchspeicheldrüse.

11. Arzneimittel zur Verwendung gemäß einem der Ansprüche 1 bis 9, welches in Kombination mit mindestens einem anti-proliferativem Mittel ausgewählt aus der Gruppe der alkylierenden Mittel, Antimetaboliten, Antimikrotubulimittel, Topoisomerase-Inhibitoren, zytotoxischen Antibiotika und Antikörper-Wirkstoff-Konjugaten zu verabreichen ist.

12. Arzneimittel zur Verwendung gemäß einem der Ansprüche 1 bis 10, welches in Kombination mit mindestens einem Antikrebs-Mittel ausgewählt aus der Gruppe der alkylierenden Mittel, Antimetaboliten, Antimikrotubulimittel, Topoisomerase-Inhibitoren, zytotoxischen Antibiotika, Antikörper-Wirkstoff-Konjugaten und/oder Strahlentherapie zu verabreichen ist.

## Revendications

1. Composition pharmaceutique comprenant un composé de formule générale (I) 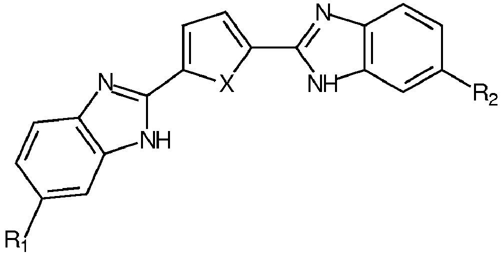
dans laquelle X est Se, O, Te, S ou NH,
et R₁ et R₂ sont identiques ou différents et sont choisis parmi 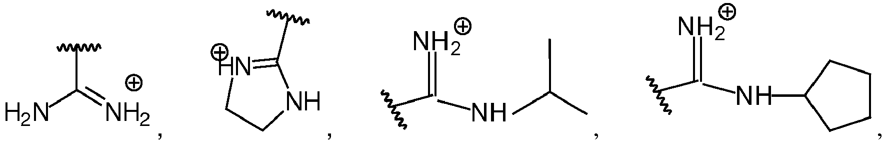 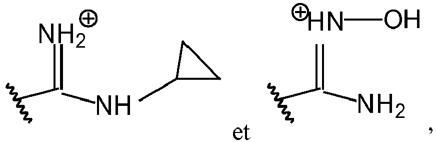
ou un sel pharmaceutiquement acceptable de celui-ci ;
comprenant éventuellement un véhicule et un ou plusieurs excipients pharmaceutiquement acceptables,
pour une utilisation dans la prophylaxie ou le traitement d'une maladie associée à une fibrose, dans laquelle la maladie est non maligne ou maligne et dans laquelle la maladie non maligne est choisie parmi une sclérose systémique (sclérodermie cutanée limitée et sclérodermie cutanée diffuse), une fibrose pulmonaire, une cirrhose hépatique, une stéatohépatite non alcoolique (NASH), une stéatohépatite alcoolique, une stéatose hépatique non alcoolique (NAFLD), une fibrose rénale, une fibrose tubulo-interstitielle, une glomérulosclérose, une sclérose vasculaire, une réaction chronique du greffon contre l'hôte, une maladie de Crohn, une recto-colite hémorragique, une colite collagène, une arthrofibrose, une myélofibrose, une maladie de Dupuytren, une fibrose systémique néphrogénique, une athérosclérose, une resténose, une fibrose cardiaque, une hypertension pulmonaire, une fibrose musculaire, une ostéoporose, une cicatrisation excessive, un développement de cicatrice chéloïde et hypertrophique, une morphée, une dégénérescence maculaire, une rétinopathie rétinienne et vitrée, et une cicatrisation oculaire, et la maladie maligne est choisie parmi un carcinome mammaire, un adénocarcinome de l'endomètre, une tumeur séreuse ovarienne, un adénocarcinome pulmonaire, une tumeur pulmonaire à cellules squameuses, une tumeur colorectale, une tumeur pancréatique, un cancer du poumon non à petites cellules (NSCLC), un carcinome à cellules squameuses, un carcinome à cellules basales, un carcinome gastrique de type intestinal, un carcinome gastrique de type diffus (mucineux), un adénocarcinome (du côlon), un carcinome hépatocellulaire, un carcinome à cellules rénales, un carcinome endométrioïde de l'endomètre, un carcinome invasif du sein, une métastase de carcinome, un cancer de la prostate et un cancer de la thyroïde.

2. Composition pharmaceutique pour une utilisation selon la revendication 1, comprenant un composé de formule générale (I) 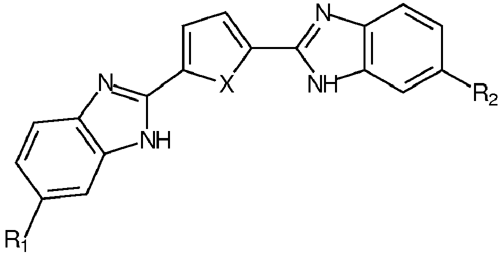
dans laquelle X est Se ou O,
et R₁ et R₂ sont identiques ou différents et sont choisis parmi 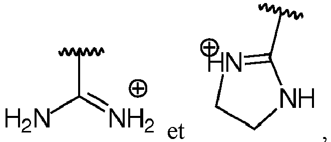
ou un sel pharmaceutiquement acceptable de celui-ci ;
comprenant éventuellement un véhicule et un ou plusieurs excipients pharmaceutiquement acceptables.

3. Composition pharmaceutique pour une utilisation selon la revendication 1 ou 2, dans laquelle X est Se.

4. Composition pharmaceutique pour une utilisation selon les revendications 1 à 3, dans laquelle R₁ = R₂.

5. Composition pharmaceutique pour une utilisation selon les revendications 1 à 4, dans laquelle X est Se et R₁ et R₂ sont tous deux 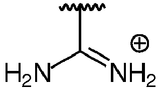 (composé DB 1976).

6. Composition pharmaceutique pour une utilisation selon les revendications 1 à 4, dans laquelle X est Se et R₁ et R₂ sont tous deux 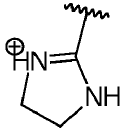 (composé DB 1977).

7. Composition pharmaceutique pour une utilisation selon l'une quelconque des revendications 1 à 6, pour une administration orale, topique ou parentérale.

8. Composition pharmaceutique pour une utilisation selon l'une quelconque des revendications 1 à 7, dans laquelle la maladie est non maligne.

9. Composition pharmaceutique pour une utilisation selon l'une quelconque des revendications 1 à 8, dans laquelle la maladie est choisie parmi une sclérose systémique (sclérodermie cutanée limitée et sclérodermie cutanée diffuse), une fibrose pulmonaire, une cirrhose hépatique, une fibrose rénale, une réaction chronique du greffon contre l'hôte, une maladie de Crohn, une arthrofibrose, une myélofibrose, une maladie de Dupuytren et une fibrose systémique néphrogénique.

10. Composition pharmaceutique pour une utilisation selon l'une quelconque des revendications 1 à 7, dans laquelle la maladie est choisie parmi un carcinome mammaire, un adénocarcinome de l'endomètre, une tumeur séreuse ovarienne, un adénocarcinome pulmonaire, une tumeur pulmonaire à cellules squameuses, une tumeur colorectale et une tumeur pancréatique.

11. Composition pharmaceutique pour une utilisation selon l'une quelconque des revendications 1 à 9, qui est destinée à être administrée en combinaison avec au moins un agent antiprolifératif choisi dans le groupe des agents d'alkylation, des antimétabolites, des agents anti-microtubules, des inhibiteurs de topoisomérase, des antibiotiques cytotoxiques, et des conjugués anticorps-médicament.

12. Composition pharmaceutique pour une utilisation selon l'une quelconque des revendications 1 à 10, qui est destinée à être administrée en combinaison avec au moins un agent anticancéreux choisi dans le groupe des agents d'alkylation, des antimétabolites, des agents anti-microtubules, des inhibiteurs de topoisomérase, des antibiotiques cytotoxiques, des conjugués anticorps-médicament, et/ou une radiothérapie.
